(19) Europäisches Patentamt
European Patent Office
Office européen des brevets

(11) Veröffentlichungsnummer: **0 472 800 A1**

(12) # EUROPÄISCHE PATENTANMELDUNG

(21) Anmeldenummer: **90810660.2**

(51) Int. Cl.[5]: **C07D 213/80**, A61K 31/455

(22) Anmeldetag: **30.08.90**

Die Bezeichnung der Erfindung wurde geändert (Richtlinien für die Prüfung im EPA, A-III, 7.3).

(43) Veröffentlichungstag der Anmeldung:
**04.03.92 Patentblatt 92/10**

(84) Benannte Vertragsstaaten:
**AT BE DE DK ES FR GB GR IT LU NL SE**

(71) Anmelder: **Sandmeier, Peter, Dr.**
**Multenweg 6**
**CH-4102 Binningen(CH)**

(72) Erfinder: **Sandmeier, Peter, Dr.**
**Multenweg 6**
**CH-4102 Binningen(CH)**

(74) Vertreter: **Frei, Alexandra Sarah**
**Frei Patentanwaltsbüro Hedwigsteig 6**
**Postfach 95**
**CH-8029 Zürich(CH)**

(54) **Metoprololnicotinsäureester, Verfahren zur Herstellung und pharmazeutische Verwendung.**

(57) Verbindungen der Formel

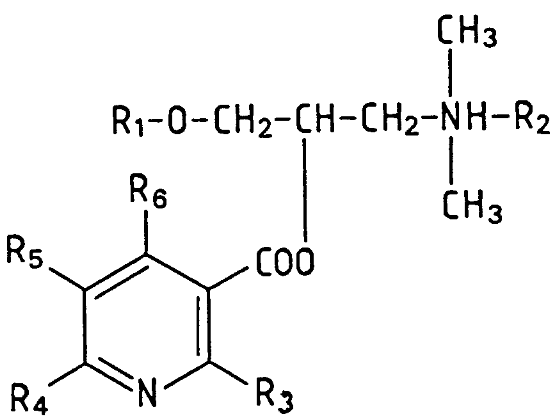

werden beschrieben, worin:
$R_1$ ein gegebenenfalls substituierter mono-, di- oder trizyklischer aromatischer Rest ist,
$R_2$ Wasserstoff oder ein Methylrest ist und $R_3$, $R_4$, $R_5$ und $R_6$ gleich oder verschieden sind und Wasserstoff, Alkyl- oder Niederalkohol-Reste sind.

Die erhaltenen Verbindungen erweisen sich als wirksame Betarezeptor-Blocker, mit verminderten Nebenwirkungen einer herabgesetzten peripheren Durchblutung bzw. eines zu starken, kurzzeitigen Abfalls des Blutdrukkes. Ein Verfahren zur Herstellung einer der erfindungsgemässen Verbindungen wird angegeben.

EP 0 472 800 A1

Die Erfindung liegt im Gebiet der pharmazeutisch wirksamen Verbindungen und betrifft Verbindungen mit Wirkung als Betarezeptorenblocker, Verfahren zu deren Herstellung sowie die Verwendung als Arzneimittel.

Die Wirkung von Arzneimitteln hängt neben ihrer spezifischen Wirkung und der Dosierung auch von der Dosierungsdynamik (Pharmakodynamik) ab. Die Dosierungsdynamik drückt sich aus in der Raschheit, mit der bestimmte Blutspiegel erzielt werden können. Oral applizierte Arzneimittel weisen damit eine ganz andere Dosierungsdynamik auf, als intravenös applizierte Arzneimittel. Die Steuerung dieser Dynamik, also die Arzneimittelkonzentration in Funktion der Zeit ist eine zentrale Forderung in der medizinischen Handhabung pharmakologisch wirksamer Moleküle. So werden dafür alle möglichen Mechanismen herangezogen, von der Verzögerung der Wirkstoffabgabe und der Verlängerung der Wirkungsdauer durch entsprechende galenische Formulierungen (Retardformulierung) bis hin zur intravenösen Applikation oder Inhalation mit raschem Konzentrationsaufbau und kurz anhaltendem maximalen Spiegel.

Für viele Medikamente wird die erwünschte Dynamik nicht erreicht, das heisst, die bekannten Massnahmen sind nicht geeignet, den angestrebten Konzentrationsverlauf herbeizuführen.

Im Falle von Betarezeptorenblockern, umgangssprachlich auch Betablocker genannt, wäre erwünscht, dass der Pegel sukzessive und relativ sanft ansteigt und möglichst lange erhalten bleibt, also langsam absinkt. Neben einer möglichst markant verlängerten Halbwertszeit, mit dem Ziel die Einnahmefrequenz niedrig zu halten, sollen auch bekannte Nebenwirkungen der Betablocker adäquat kompensiert werden. Die Applikation soll vorzugsweise oral erfolgen.

Es ist das Ziel der Erfindung, eine Verbindung zu finden, die diese Anforderungen erfüllt.

Es gibt Verbindungen, sogenannte Prodrugs, die nach Resorption im Körper in die wirksame Form umgewandelt werden. Auf diese Weise ist es möglich, die wirksame Form mit geringerem Wirksamkeitsverlust in das Zielkompartiment, meistens das Plasma, einzubringen. Der Vorteil besteht in einer starken Verringerung der Verlustdosis, das ist der mengenmässige Anteil an biologisch verfügbarem Wirkstoff, der zwischen Start- (Ort der Applikation) und Zielkompartiment (Plasma) verloren geht. Ein weiterer Vorteil besteht darin, dass für das Erzielen eines wirksamen Plasmaspiegels unter Umständen eine geringere Dosis ausreicht, was die Verringerung der dosisabhängigen unerwünschten Wirkungen nach sich zieht.

Es ist also vorteilhaft, nach Verbindungen zu suchen, die auf diesem Weg im Körper wirksam werden.

Die neuen erfindungsgemässen Verbindungen haben die allgemeine Formel,

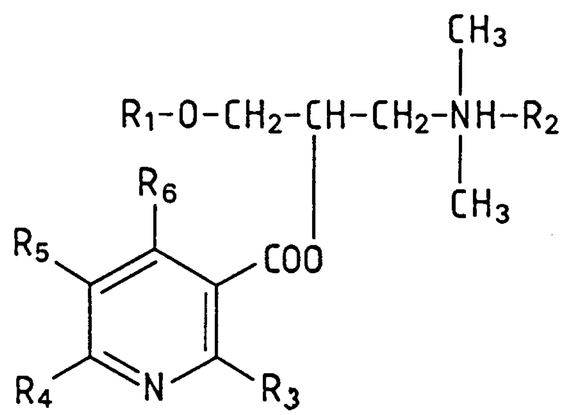

I

worin

$R_1$ ein gegebenenfalls substituierter mono-, di- oder trizyklischer aromatischer Rest ist,

$R_2$ Wasserstoff oder ein Methylrest ist und $R_3$, $R_4$, $R_5$ und $R_6$ gleich oder verschieden sind und Wasserstoff, alkyl- oder niederalkohol-Reste sind.

Bevorzugt sind Verbindungen, worin $R_1$ eine 4-Methoxyethylphenoxymethylgruppe oder eine 1H-Indol-4-yloxymethylgruppe oder eine 2-hydroxy-3-isopropylamino-propoxyphenylgruppe ist.

Spezifische Beispiele von Verbindungen der Formel I haben folgende Gruppe von mono-, di- oder trizyklischer aromatischer Resten als $R_1$ und folgende Gruppe eines Wasserstoff- oder Methylrests als R2:

| | |
|---|---|
| 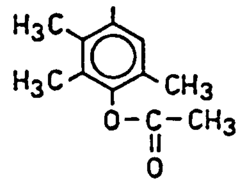 | -H |
| 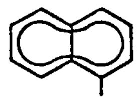 | -H |
| 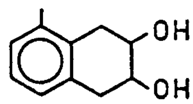 | $-CH_3$ |
| 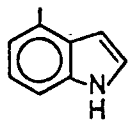 | -H |
| 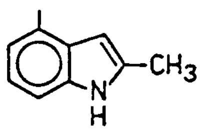 | -H |
| 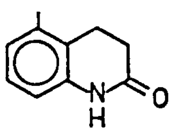 | $-CH_3$ |
| 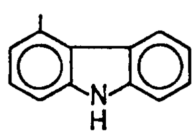 | -H |
| 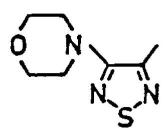 | $-CH_3$ |

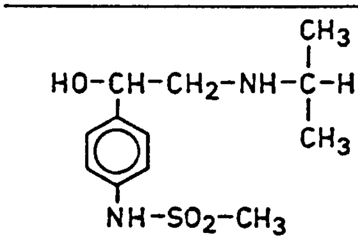

| | |
|---|---|
| 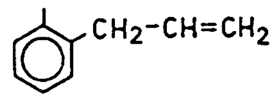 | -H |
| 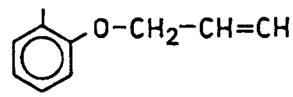 | -H |
| 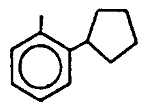 | $-CH_3$ |
| 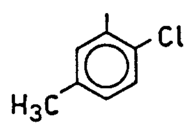 | $-CH_3$ |
| 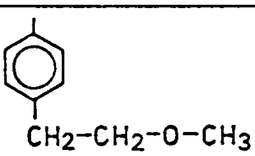 | -H |
| 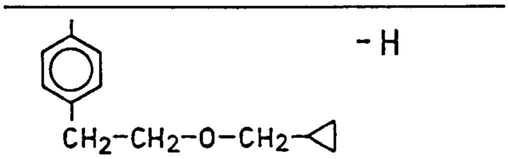 | -H |
| 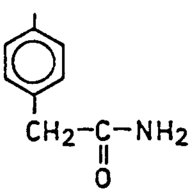 | -H |
| 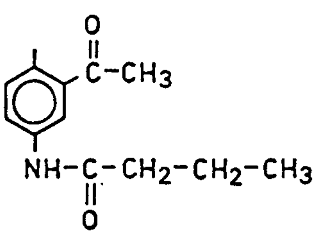 | -H |

Verbindungen dieser Art sind Prodrugs, welche erst nach der Resorption im Körper durch hydrolytische Spaltung in eine Säure und einen Betabloker gespalten werden. Daraus ergeben sich gegenüber den konventionellen Betablockern folgende Vorteile:

- Kein scharfer Anstieg der Blutspiegelwerte und als Konsequenz davon kein scharfer Abfall des Blutdruckes. Der Verzögerungsmechanismus ist hier die notwendige Hydrolyse des Moleküls in mindestens einen klinisch wirksamen Betandteil.
- Eine verlängerte Halbwertszeit, also eine chemische Retardierung. Als direkte Konsequenz davon, sind nur noch 1 bis 2 malige Gaben/24h nötig, was gerade bei Bluthochdruck wichtig ist, da dieser eine Dauertherapie für den Rest des Lebens erfordert (bessere compliance oder Akzeptanz durch den Patienten). Der Retardierungsmechanismus ist hier ebenfalls die langsam ablaufende Hydrolyse.
- Es hat sich ferner gezeigt, dass (reproduzierbar) wesentlich niedrigere Dosierungen verabreicht

werden können (im Tierversuch ungefähr 10%-50% der üblichen Dosierung), um den gleichen therapeutischen Effekt zu erzielen. Der Mechanismus dafür kann im Moment nicht erklärt werden. Vermutlich ist die Reduktion proportional zur Verlustmenge für die Überwindung der Resorptionsbarriere.

Wie oben schon dargelegt, ist eines der Hydrolyseprodukte des erfindungsgemässen Prodrugesters ein Betablockermolekül. Das andere Hydrolyseprodukt ist vorzugsweise ein Molekül, das fähig ist, die Nebenwirkungen eines Betablockers zu reduzieren. Diese Nebenwirkungen sind folgende:

- Betablocker führen in der Regel zu einer Verschlechterung der peripheren Durchblutung. Dies ist deswegen nicht überraschend, weil diese Substanzen die physiologische Weitstellung sowohl der peripheren Widerstands- als auch der Kapazitätsgefässe blockieren. Der gleiche Mechanismus, der zu einer gewünschten Blutdrucksenkung führt, bewirkt auch die unerwünschte Verschlechterung der peripheren Durchblutung.

  Dieser Mechanismus darf, auch aufgrund der negativen Nebenwirkungen chemisch nicht beeinflusst (angegriffen) sondern nur kompensiert werden. Ein Kompensationsmechanismus ist, die periphere Durchblutung zu verbessern ohne den Blutdruck erhöhen zu müssen. Dieses Ziel wird erreicht, wenn die erfindungsgemässe Verbindung, also die Prodrug, aus einem Betablocker und einer Substanz besteht, die die peripheren Gefässe direkt erweitert. In einem solchen Fall wären die beiden Hydrolyseprodukte bezüglich der Blutdrucksenkung Synergisten.

Erfindungsgemäss wird daher vorgeschlagen, den Säureanteil des erfindungsgemässen Prodrug durch Nikotinsäure zu realisieren. Das Hydrolyseprodukt Nicotinsäure hat im übergeordneten pharmazeutischen Mechanismus folgende Vorteile:

- Die freie Nikotinsäure bewirkt eine Erweiterung des Kapillarnetzes was die Durchströmung desselben bei geringerem Blutdruck verbessert. Der Betablocker, mit dem der Blutdruck abgesenkt werden soll und auch wird, und deswegen eine schlechtere Durchströmung (lediglich mangels Druck) bewirkt, wird durch die Erweiterung des Kapillarnetzes nicht in seiner Wirkung (Blutdrucksenkung) beeinträchtigt, aber die Wirkung wird durch die Erweiterung des Kapillarnetzes gleichsam entschärft.
- Ferner ist bekannt, dass die Nikotinsäure erhöhte Cholesterin- und Triglycerid-Werte, die bei Patienten mit Bluthochdruck meistens zu verzeichnen sind, senkt und damit eine erwünschte Nebenwirkung aufweist.
- Letzlich wird die Nicotinsäure wie der Betablocker infolge der vorgängig notwendigen Hydrolyse verzögert freigesetzt, was die bekannte rasche Gefässerweiterung ("Flush") bei schnellem Wirkungseintritt verhindert.

Das erfindungsgemässe Molekül bietet nicht nur die Vorteile einer herkömmlichen Prodrug, nämlich die Retardierung und eventuelle Verbesserung der Resorption, sondern auch weitere Vorteile: Die beiden Molekülteile, die durch Hydrolyse freigesetzt werden, unterstützen sich bezüglich ihrer Hauptwirkung derart, dass das Auftreten von bestimmten unerwünschten Nebenwirkungen der einzelnen Substanzen (hier die Verschlechterung der peripheren Durchblutung durch Betablocker und die Flushwirkung durch die Nicotinsäure) durch die andere kompensiert wird, und dass die Dosis des Betablokers auf 10-50% der üblichen Dosis gesenkt werden kann. Die betreffenden Mechanismen sind oben bereits dargestellt. Das vorgeschlagene neue Molekül gehört damit nicht nur zu den Prodrugs, sondern auch zu den Kombinationspräparaten. Damit gehört es zu einer neuen Familie von spaltbaren Molekülen mit dem charakteristischen Merkmal, dass die Hydrolyseprodukte bspw. die Hauptnebenwirkung des Partners abschwächen bis kompensieren und so ein Zusammenwirken der Eigenschaften (Faktoren) der einzelnen Komponenten zu einer abgestimmten Gesamtleistung ermöglichen, die mit einer lediglichen Mischung der einzelnen Elemente (Kombination nicht als Prodrug) nicht erreicht werden kann, bspw. der zeitlich abgestimmte Zerfall durch Hydrolyse im gleichen Zielkompartiment. Das neue Molekül ist auf Toxizität geprüft und als gut befunden worden, bis jetzt konnten keine unerwünschten Nebenwirkungen festgestellt werden. Damit kommt das neue Molekül pharmakologisch einer Mischung der einzelnen Elemente nahe, allerdings mit den geschilderten grossen Vorteilen.

Eine solche Gesamtwirkung ist jedoch das typische Charakteristikum für den Synergismus. Synergisten sind Substanzen, die sich in ihrer Wirkung additiv oder potenzierend ergänzen. Es wird deshalb vorgeschlagen, diese neue Familie "präsynergische Verbindungen" zu nennen.

Ein Beispiel für eine Anzahl solcher präsynergischer Verbindungen ist in der Formel I gezeigt. Sie besitzen in der $\beta$-Position zum Sauerstoffatom der Säure ein assymmetrisches Kohlenstoffatom des Betablockermoleküls. Bei der Herstellung führt dies zu einem racemischen Gemisch von optisch aktiven Isomeren von R- und S-enantiomorphen Verbindungen, die mit bekannten Methoden getrennt und gereinigt werden können. Bevorzugt sind hier die S-enantiomorphen Verbindungen. Freie Basen der Verbindungen gemäss der allgemeinen Formel I, können in Säureadditionssalze, bzw. die Säureadditionssalze in freie

Basen (ineinander) umgewandelt werden. Diese Vorgehen sind an sich bekannt.

Die Ausgangsverbindungen (Säure und Betablocker) sind entweder bekannt oder sie können durch bekannte Verfahren hergestellt werden. Gemäss der dargelegten Erfindung ist es möglich, synergistisch wirkende Paare von Ausgangsstoffen auszuwählen und zu einem Prodrug bzw. zu einem "Präsynergicum" synthetisieren.

In Form freier Basen oder in Form von Säureadditionssalzen können diese Substanzen in verschiedenen pharmazeutischen Darreichungsformen wie Tabletten, Schichttabletten, Kapseln oder in flüssiger Form angeboten werden. Verbindungen der Formel I wirken biologisch als Betarezeptorenblocker und können in der Prophylaxe und der Behandlung von Koronarkrankheiten, bspw. Angina pectoris angewendet werden.

Nachfolgend ist ein Beispiel zur Herstellung eines erfindungsgemässen Moleküls mit Metoprolol als Betablocker als dem einen und Nicotinsäure als dem anderen Synergisten beschrieben. Es wird davon ausgegangen, dass die anderen, oben angegebenen Betablocker sich ebenfalls zu einem Prodrug verbinden lassen:

Ein Verfahren zur Herstellung von Verbindungen der Formel I in dem man eine Verbindung der Formel

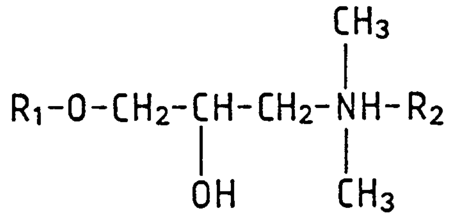

II

mit einer Verbindung der Formel III oder einem reaktionsfähigen Derivat davon verestert,

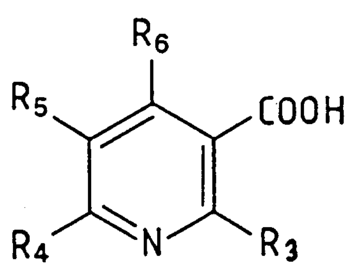

III

welche Verbindung eine Säure ist und deren Stickstoffatom gegebenenfalls geschützt ist.

Eine Mischung aus 19,8 g Metoprololtartrat und 12,8 g $(BOC)_2O$ in 80 ml tert-Butanol und 1,80 g NaOH wird während 24 Stunden bei Raumtemperatur gerührt. Es werden 21,3 g BOC-Metoprolol erhalten.

Diese Menge BOC-Metoprolol wird in 100 ml Methylenchlorid gelöst und 19,0 g Nicotinylchlorid HCl zugegeben. Die Mischung wird während 2 Std. im Rückfluss erwärmt. Es werden 21,75 g BOC-Metoprololnicotinsäure-Ester erhalten.

Diese Menge des gewonnenen Esters wird in 200 ml 90%-Fumarsäure aufgelöst und während 25 Minuten bei 60° C gerührt. Nach dem Abdampfen der überschüssigen Fumarsäure, wird der Rückstand in 100 ml Toluol gelöst und nochmals abgedampft. Damit sollte keine überschüssige Fumarsäure mehr im Rückstand sein.

Der nun vorliegende Rückstand wird nun mit 200 ml eines 1:1 Gemisches von Chloroform und Diisopropyläther versehen und 46 ml 1N HCl beigefügt. Die Mischung wird wieder abgedampft und der Rückstand mit Chloroform verdünnt und über $NaSO_4$ sicc. getrocknet.

Das Endprodukt ist ein gelbfarbenes Oel, das nach ungefähr 3 Tagen bei Raumtemperatur auszukristallisieren beginnt. Um ein reines Präparat zu erhalten, wird in Diisopropyläther rekristallisiert.

| | |
|---|---|
| Ausbeute: | 14,10 g Metoprololnicotinsäureester-HCl gemäss Formel = 76% |
| Schmelzpunkt: | 100 - 102° C |
| Molekulargewicht: | 408,93 g/mol |
| theoretisch erreichbar: | C 61,68% H 7,15% N 6,85% $H_2O$ |

realisiert wurde: C 61,56% H 7,07% N 7,34% $H_2O$
Dünnschichtchromatographie in Butanol/AcOH/$H_2O$ 4/1/1 Rf = 0,22

Analog können mit dem gleichen Vorgehen, nämlich Wahl eines Betablockers mit Schutzgruppe, Zugabe von Nicotinylchlorid und Erwärmen, den gebildeten Ester in Fumarsäure auflösen und wärmen und den freien Ester isolieren, eine grosse Zahl von Betablockern kombiniert werden.

Die verhältnismässig einfache Synthese führt zu einer bemerkenswert hohen Ausbeute des erfinderischen Moleküls, der Metoprololnicotinsäure. Die an Hunden experimentierte Prodrugwirkung in drei verschiedenenen Dosen ergab folgende Retardwerte: (µg freie Base im Zielkompartiment)

| | | | | | | | |
|---|---|---|---|---|---|---|---|
| Std | 0 | 0.5 | 1.0 | 2.0 | 4.0 | 6.0 | 8.01224 |
| A | 0 | 11.0 | 18.9 | 13.9 | 13.5 | 8.2 | 3.26.40 |
| B | 0 | 6.8 | 40.7 | 53.3 | 31.9 | 34.1 | 26.77.50.8 |
| C | 0 | 2.6 | 4.7 | 4.5 | 2.9 | 0.9 | 0.70.45.9 |

Das Resultat zeigt, dass eine relativ hohe Konzentration im Plasma bis zu vier Stunden erwartet werden kann, was die deutlich retardierte Prodrugwirkung beweist. Die Dosis C zeigt unter anderem, dass auch kleine Konzentrationen einen langanhaltenden Spiegel zeigen.

## Patentansprüche

**1.** Verbindung der Formel

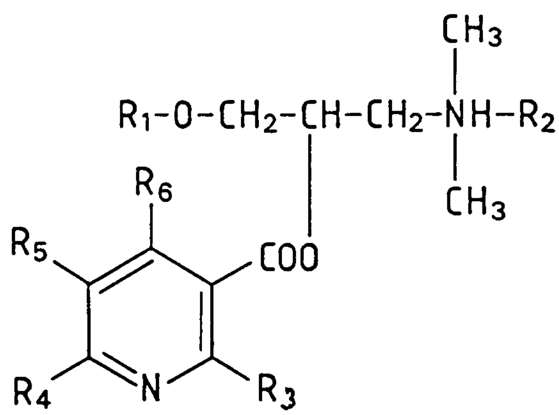

worin

$R_1$ ein gegebenenfalls substituierter mono-, di- oder trizyklischer aromatischer Rest ist,

$R_2$ Wasserstoff oder ein Methylrest ist und $R_3$, $R_4$, $R_5$ und $R_6$ gleich oder verschieden sind und Wasserstoff, Alkyl- oder Niederalkohol-Reste sind.

**2.** Verbindungen nach Anspruch 1, dadurch gekennzeichnet, dass $R_1$ eine 4-Methoxyethylphenoxymethylgruppe ist.

**3.** Verbindungen nach Anspruch 1, dadruch gekennzeichnet, dass $R_1$ eine eine 1H-Indol-4-yloxymethylgruppe ist.

**4.** Verbindungen nach Anspruch 1, dadruch gekennzeichnet, dass $R_1$ eine 2-hydroxy-3-isopropylaminopropoxyphenylgruppe ist.

**5.** Verbindung nach Anspruch 1, dadurch gekennzeichnet, dass sie aus durch Hydrolyse trennbarem Alkoholteil und Säureteil besteht, die beide gesondert eine spezifisch pharmazeutische Wirkung haben,

die je einen Wirkungsanteil der anderen Komponente wirkungsmässig neutralisiert.

6. Verbindung nach Anspruch 5, dadurch gekennzeichnet, edass der Alkoholteil ein β-Rezeptor-Blocker und der Säureteil ein gefässerweiterndes Mittel ist.

7. Verbindung nach Anspruch 6, dadurch gekennzeichnet, dass der β-Blocker Metoprolol, Pindolol, Atenolol oder Propranolol ist und das gefässerweiternde Mittel Nicotinsäure ist.

8. Verfahren zur Herstellung von Verbindungen der Formel I nach Anspruch 1 oder 2, dadurch gekennzeichnet, dass die Verbindungen mittels an und für sich bekannten Verfahren herstellbar sind.

9. Verfahren zur Herstellung von Verbindungen der Formel I, dadurch gekennzeichnet, dass man eine Verbindung der Formel

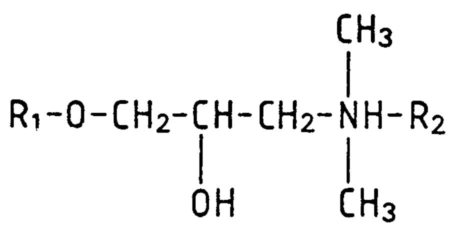

mit einer Verbindung der Formel III oder einem reaktionsfähigen Derivat davon verestert,

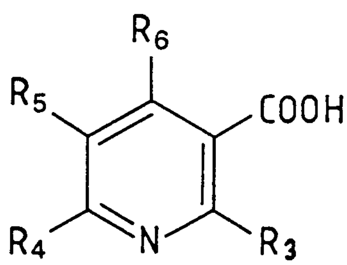

welche Verbindung eine Säure ist und deren Stickstoffatom gegebenenfalls geschützt ist, wobei $R_1$ bis $R_6$ die in Anspruch 1 angegebene Bedeutung aufweisen können.

10. Arzneimittel, enthaltend wenigstens eine Verbindung der Formel I, wie in Anspruch 1 definiert, zusammen mit einem pharmazeutischen Trägermittel.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung
EP 90 81 0660

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | FR-A-2 254 315 (PRODUITS CHIMIQUES UGINE KUHLMANN)<br>* Das ganze Dokument *<br>-- | 1,5, 6,8-10 | C 07 D 213/80<br>A 61 K 31/455 |
| A | CHEMICAL ABSTRACTS, Band 107, 1987, Seite 604, Zusammenfassung Nr. 115352m, Columbus, Ohio, US; & CN-A-85 102 267 (FAMING ZHUANLI SHENQING GONGKAI SHUOMINGSHU) 10-11-1985<br>* Zusammenfassung *<br>-- | 1,10 | |
| X | DE-A-2 331 721 (SANDOZ)<br>* Beispiel 10; Seite 11 *<br>---- | 1,10 | RECHERCHIERTE SACHGEBIETE (Int. Cl.4)<br>C 07 D 213/00<br>A 61 K 31/00 |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche:
Unvollständig recherchierte Patentansprüche: 1-10
Nicht recherchierte Patentansprüche:
Grund für die Beschränkung der Recherche:

Siehe Blatt -C-

| Recherchenort | Abschlußdatum der Recherche | Prüfer |
|---|---|---|
| DEN HAAG | 12-04-1991 | DE JONG |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1505.1 03.82

EP 90 81 0660 -C-

Die Markushformel in Anspruch 1 und die erste Markushformel in Anspruch 9 sind falsch: Sie enthalten ein Stickstoffatom, das verbunden ist mit 5 anderen Atomen.
Die Recherche ist deshalb begrenzt auf Verbindungen mit Markushformel A.

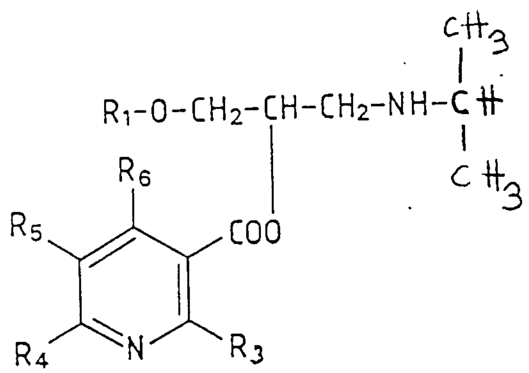

A

Für die bedeutung von $R_1,R_3,R_4,R_5,R_6$ siehe Anspruch 1 der Anmeldung.